# EUROPÄISCHE PATENTSCHRIFT

(11) **EP 2 558 458 B1**
(45) Veröffentlichungstag und Bekanntmachung des Hinweises auf die Patenterteilung: **06.09.2017**
(21) Anmeldenummer: 11713792.7
(22) Anmeldetag: 11.04.2011
(51) Int. Cl.: C07D 401/04, C07D 417/04, C07D 417/14, A01N 43/56, A01N 43/78

(54) **NEUE HETEROCYCLISCHE VERBINDUNGEN ALS SCHÄDLINGSBEKÄMPFUNGSMITTEL**
NOVEL HETEROCYCLIC COMPOUNDS AS PEST CONTROL AGENTS
NOUVEAUX COMPOSÉS HÉTÉROCYCLIQUES EN TANT QU'AGENTS ANTIPARASITAIRES

(30) Priorität: 16.04.2010 US 325094 P; 16.04.2010 EP 10160189
(43) Veröffentlichungstag der Anmeldung: 20.02.2013
(73) Patentinhaber: Bayer Intellectual Property GmbH, 40789 Monheim (DE)
(72) Erfinder: BRETSCHNEIDER, Thomas, 53797 Lohmar (DE); KÖHLER, Adeline, 40764 Langenfeld (DE); FISCHER, Reiner, 40789 Monheim (DE); FÜßLEIN, Martin, 40225 Düsseldorf (DE); JESCHKE, Peter, 51467 Bergisch Gladbach (DE); KLUTH, Joachim, 40764 Langenfeld (DE); MÜHLTHAU, Friedrich August, 65812 Bad Soden am Taunus (DE); VOERSTE, Arnd, 50674 Köln (DE); MALSAM, Olga, 51503 Rösrath (DE); GÖRGENS, Ulrich, 40882 Ratingen (DE); SATO, Yoshitaka, Tsukuba-shi, Ibaraki 3002648 (JP)
(74) Vertreter: BIP Patents
(86) Internationale Anmeldenummer: PCT/EP2011/055645
(87) Internationale Veröffentlichungsnummer: WO 2011/128304

(56) Entgegenhaltungen:
- WO-A1-98/57969
- WO-A1-03/044000
- WO-A1-2004/103980
- WO-A1-2009/149858
- DE-A1- 2 221 647
- DE-A1- 2 704 288
- DATABASE REGISTRY [Online] CHEMICAL ABSTRACTS SERVICE, COLUMBUS, OHIO, US; 13. Oktober 2003 (2003-10-13), XP002604750, gefunden im STN Database accession no. 603073-25-2(RN)
- DATABASE REGISTRY [Online] CHEMICAL ABSTRACTS SERVICE, COLUMBUS, OHIO, US; 26. Juni 2007 (2007-06-26), XP002604751, gefunden im STN Database accession no. 939230-00-9(RN)
- DATABASE REGISTRY [Online] CHEMICAL ABSTRACTS SERVICE, COLUMBUS, OHIO, US; 26. Juni 2007 (2007-06-26), XP002604752, gefunden im STN Database accession no. 939230-12-3(RN)
- DATABASE REGISTRY [Online] CHEMICAL ABSTRACTS SERVICE, COLUMBUS, OHIO, US; 26. Juni 2007 (2007-06-26), XP002604753, gefunden im STN Database accession no. 939230-18-9(RN)
- DATABASE REGISTRY [Online] CHEMICAL ABSTRACTS SERVICE, COLUMBUS, OHIO, US; 19. April 2009 (2009-04-19), XP002604754, gefunden im STN Database accession no. 1136635-65-8(RN)

## Beschreibung

Die vorliegende Anmeldung betrifft neue Amide, Verfahren zu Ihrer Herstellung und ihre Verwendung zur Bekämpfung von tierischen Schädlingen, zu denen Arthropoden und insbesondere Insekten zählen.

Bestimmte Amide und Thioamide sind bereits als insektizid wirksame Verbindungen bekannt geworden (vgl. DE 2221647, WO 2009/149858, WO 2010/129497).

Ferner ist die Verbindung der Formel 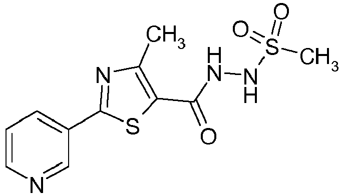 bekannt geworden (CAS Registry No. 262855-20-9), für die aber keine Verwendung beschrieben wurde.

Moderne Planzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch neue Verbindungen der Formel (I), 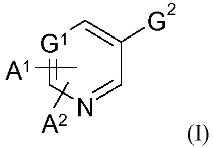 worin
- A¹: für Wasserstoff, Halogen oder Cyano steht,
- A²: für Wasserstoff steht,
- G¹: für N oder C-A¹ steht,
- G²: für einen Rest aus der Reihe 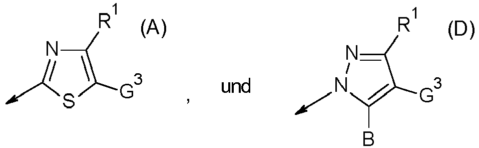 steht, worin der Pfeil die Bindung zum benachbarten Ring markiert,
- R¹: für Wasserstoff oder C₁-C₄-Alkyl steht,
- B: für Wasserstoff steht,
- G³: für den Rest 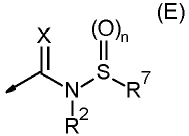 steht, worin der Pfeil die Bindung zu G² markiert,
- X: für Sauerstoff steht,
- n: für 2 steht,
- R²: für einen Rest aus der Reihe Wasserstoff, C₁-C₄-Alkyl und C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein Kation, wie beispielsweise ein ein- oder zweiwertiges Metallion oder ein gegebenenfalls durch C₁-C₄-Alkyl oder Aryl-C₁-C₄-alkyl substituiertes Ammonium-Ion steht,
- R⁷: für einen Rest aus der Reihe jeweils gegebenenfalls durch Halogen, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl, C₂-C₄-Alkinyl, jeweils gegebenenfalls durch Halogen, C₁-C₄-Alkyl, C₁-C₄-Halogenalkyl, C₁-C₄-Alkoxy, C₁-C₄-Halogenalkoxy substituiertes C₃-C₆-Cycloalkyl, C₃-C₆-Cycloalkyl-C₁-C₄-alkyl, C₃-C₄-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl und Heteroaryl-C₁-C₄-alkyl oder für NR'R" steht, worin R' und R" unabhängig voneinander für einen Rest aus der Reihe Wasserstoff und C₁-C₄-Alkyl stehen,
worin Halogen ausgewählt ist aus der Reihe Fluor, Chlor, Brom und Iod, Aryl (auch als Teil einer größeren Einheit, wie beispielsweise Arylalkyl) ausgewählt ist aus der Reihe Phenyl, Naphthyl, Anthryl, Phenanthrenyl und Hetaryl (auch als Teil einer größeren Einheit, wie beispielsweise Hetarylalkyl) ausgewählt ist aus der Reihe Pyrimidyl, Oxadiazolyl, Oxazolyl, Pyrazinyl, Imidazolyl, Thiazolyl und Furanyl,
sowie Salze und N-Oxide der Verbindungen der Formel (I).

Weiter wurde gefunden, dass man die neuen Verbindungen der Formel (I) nach dem im Folgenden beschriebenen Verfahren erhalten kann.

Verbindungen der Formal (I) können beispielsweise durch Umsetzung der heterocyclischen Carbonsäuren der Formel (II₁) oder deren Säurechloriden mit Aminderivaten der Formel (III) hergestellt werden. 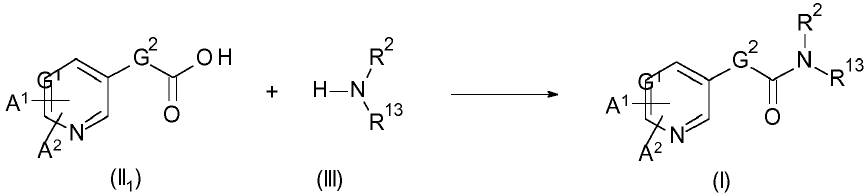 wobei R¹³ für 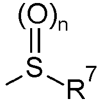 steht.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die Verbindungen der Formel (I) können gegebenenfalls in Abhängigkeit von der Art der Substituenten als geometrische und/oder als optisch aktive Isomere oder entsprechende Isomerengemische in unterschiedlicher Zusammensetzung vorliegen. Die Erfindung betrifft sowohl die reinen Isomere als auch die Isomerengemische.

Die erfindungsgemäßen Verbindungen können auch als Metallkomplexe vorliegen, wie sie für andere Amide beispielsweise in DE 2221647 beschrieben sind.

Bevorzugt sind Verbindungen der Formel (I), worin
A¹ für einen Rest aus der Reihe Wasserstoff, Fluor und Chlor steht,
A² für Wasserstoff steht,
G¹ für einen Rest aus der Reihe N, C-H, C-F und C-Cl steht,
G² für einen Rest aus der Reihe 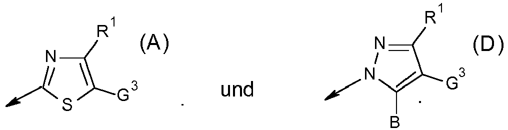 steht, worin der Pfeil die Bindung zum benachbarten Ring markiert,
R¹ für Wasserstoff oder Methyl steht,
B für Wasserstoff steht,
G³ für den Rest 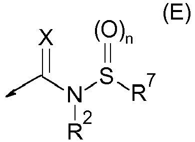 steht, worin der Pfeil die Bindung zu G² markiert,
X für Sauerstoff steht,
n für 2 steht,
R² für einen Rest aus der Reihe Wasserstoff, Methyl, Ethyl, CH₂OCH₃, CH₂OCH₂CH₃, COCH₃, COCH₂CH₃, Cyclopropyl, Na⁺, K⁺ und ⁺NMe₄ steht und
R⁷ für einen Rest aus der Reihe Methyl, Ethyl, i-Propyl, CF₃, CHF₂, CH₂F, CH₂CF₃, Cyclopropyl, Dimethylamino, Diethylamino, Phenyl und Benzyl steht.

Durch Halogen substituierte Reste, z.B. Haloalkyl (= Halogenalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

In den Resten (A) und (D), für die G² stehen kann, markiert der Pfeil jeweils die Bindung zum benachbarten Ring.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend.

In einer hervorgehobenen Gruppe von erfindungsgemäßen Verbindungen steht G² für den Rest (A).

In einer weiteren hervorgehobenen Gruppe von erfindungsgemäßen Verbindungen steht G² für den Rest (D).

In einer weiteren hervorgehobenen Gruppe von erfindungsgemäßen Verbindungen steht G¹ für C-H.

In einer weiteren hervorgehobenen Gruppe von erfindungsgemäßen Verbindungen steht G¹ für C-F.

In einer weiteren hervorgehobenen Gruppe von erfindungsgemäßen Verbindungen steht G¹ für Stickstoff.

In einer weiteren hervorgehobenen Gruppe von erfindungsgemäßen Verbindungen steht A¹ für Wasserstoff.

In einer weiteren hervorgehobenen Gruppe von erfindungsgemäßen Verbindungen steht R¹ für Wasserstoff.

In einer weiteren hervorgehobenen Gruppe von erfindungsgemäßen Verbindungen steht R¹ für Methyl.

Die Herstellung erfindungsgemäßer Verbindungen der Formel (I), in welcher G² für den Rest (A) steht, und entsprechender Vorstufen werden im folgenden Reaktionsschema erläutert. 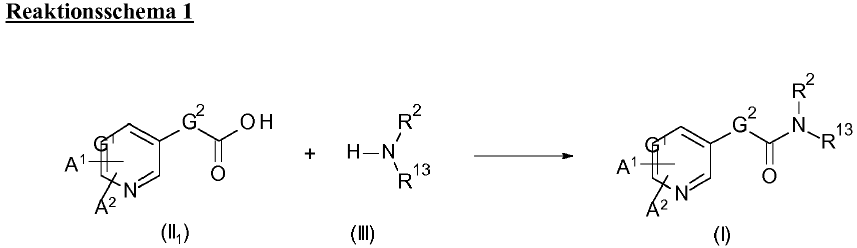 wobei R¹³ für 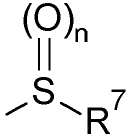 steht.

Verbindungen der Formel (I) können beispielsweise hergestellt werden durch Umsetzung der heterocyclischen Carbonsäuren der Formel (II₁) oder der entsprechenden Säurechloride mit Aminderivaten der Formel (III).

Die als Ausgangsstoffe benötigten Säuren der Formel (II₁), in welcher G² für den Reste (A) steht, können analog zu den Methoden hergestellt werden, die in WO 2009/149858 beschrieben sind.

Die als Ausgangsstoffe benötigten Säuren der Formel (II₁), in welcher G² für den Rest (D) steht, können analog zu den für G¹ gleich CH und R¹ gleich H in Journal of Heterocyclic Chemistry 1981, 18, 9-14 und Journal of Organic Chemistry 2004, 69, 5578-5587 beschriebenen Methoden hergestellt werden.

Die als Ausgangstoffe benötigten Aminderivaten der Formel (III) sind bekannt oder lassen sich nach im Prinzip bekannten Methoden herstellen.

Die Säuren der Formel (II₁) lassen sich nach Aktivierung, beispielsweise zum Säurechlorid (siehe beispielsweise Bioorg & MedChem Letters 15, 4354 (2005)), oder mittels Aktivierungsreagenzien wie CDI (Carbonyldiimidazol, siehe beispielsweise Bioorg & MedChem 9, 1543 (2001), EDC (1-Ethyl-3-[3-dimethylaminopropyl]carbodiimidehydrochlorid) in Gegewart von DMAP (Dimethylaminopyridin, siehe beispielsweise J. Med. Chem. 50, 3101 (2007)), oder DCC (Dicyclohexylcarbodiimid) in Gegenwart von HOBT (1-Hydroxybenztriazol, siehe beispielsweise J.Med.Chem. 50, 3101 (2007)), mit Sulfonamiden der Formel (III), gegebenenfalls in Gegenwart einer Base wie einem Metallhydrid (insbesondere Natriumhydrid) oder DBU (Diazabicycloundecen) zu den erfindungsgemäßen Verbindungen der Formel (I) umsetzen.

N-Oxide lassen sich beispielsweise durch Umsetzung von Verbindungen der Formel (I) mit mCPBA (meta-Chlorperbenzoesäure) gewinnen. Salze von Verbindungen der Formel (I) sind zugänglich, indem man Verbindungen der Formel (I) mit Verbindungen der Formel RX umsetzt, in der X beispielsweise für Halogen wie Chlor oder Brom und R beispielsweise für einen jeweils gegebenenfalls substituierten Alkyl-, Alkenyl- oder Alkinylrest steht.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-FettalkoholEther, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, AlkylAryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können noch weitere Hilfsstoffe in den Formulierungen und den daraus abgeleiteten Anwendungsformen enthalten sein. Solche Zusatzstoffe sind beispielsweise schützende Kolloide, Bindemittel, Klebstoffe, Verdicker, thixotrope Stoffe, Penetrationsförderer, Retentionsförderer, Sequestiermittel, Komplexbildner, Humectans, Spreitmittel. Im Allgemeinen können die Wirkstoffe mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern, wie beispielsweise Dioctylsulfosuccinat, oder die die Visko-Elastizität erhöhen, wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen agrochemischer Wirkstoffe in Pflanzen zu verbessern. Penetrationsförderer werden in diesem Zusammenhang dadurch definiert, dass sie aus der (in der Regel wässrigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate, wie beispielsweise Kokosfettethoxylat (10) oder Isotridecylethoxylat (12), Fettsäureester wie beispielsweise Rapsöl- oder Sojaölmethylester, Fettaminalkoxylate, wie beispielsweise Tallow Amine Ethoxylat (15), oder Ammonium und / oder Phosphonium-Salze, wie beispielsweise Ammoniumsulfat oder Diammonium-hydrogenphosphat.

Die Formulierungen enthalten im allgemeinen zwischen 0,01 und 98 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln oder Semiochemicals vorliegen.

Auch eine Mischung mit anderen bekannten Wirkstoffen, wie Herbiziden, Düngemitteln, Wachstumsregulatoren, Safenern, Semiochemicals, oder auch mit Mitteln zur Verbesserung der Pflanzeneigenschaften ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Auf streichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c, Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec., Tryptodendron spec., Apate monachus, Bostrychus capucins, Heterobostrychus brunneus, Sinoxylon spec., Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Herstellungsbeispiele

### Beispiel D: N-(Dimethylsulfamoyl)-1-(pyridin-3-yl)-1H-pyrazol-4-carboxamid

### Stufe 1: Ethyl-1-(pyridin-3-yl)-1H-pyrazol-4-carboxylat

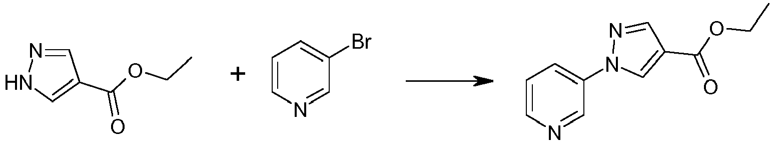

0,412 mL (4,28 mmol) 3-Brompyridin wurden in 2 mL Dimethylformamid gelöst. Anschließend wurden nacheinander 1,035 g (7,49 mmol) Kaliumcarbonat, 0,034 g (0,017 mmol) Kupferiodid, 0,081 g (0,71 mmol) Trans-N,N'-Dimethyl-1,2-cyclohexandiamin und 0,500 g (5,371 mmol) Pyrazol-4-carbonsäureethylester zugegeben. Die Mischung wurde 24 h auf 110 °C erhitzt und anschließend auf Raumtemperatur abgekühlt. Wasser wurde zugegeben und der gebildete Niederschlag wurde abfiltriert.

Ausbeute: 600 mg (77% d. Th.), logP¹⁾ (HCOOH) 1,55
¹H-NMR((CD₃)₂SO): 1,31 (t, 3H), 4,29 (q, 2H), 7,57-7,60 (m, 1H), 8,20 (s, 1H), 8,31-8,34 (m, 1H), 8,61 (bs, 1H), 9,19 (bs, 2H) ppm.

### Stufe 2: 1-(Pyridin-3-yl)-1H-pyrazol-4-carbonsäure

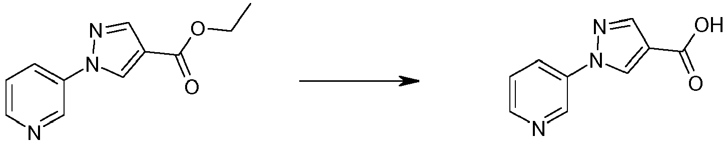

9,10 g (41,8 mmol) Ethyl-1-(pyridin-3-yl)-1H-pyrazol-4-carboxylat wurden in 170 mL Dioxan gelöst, und mit 17 mL Wasser und 8,94 g einer 45 %igen wässrigen Natriumhydroxidlösung versetzt. Die Mischung wurde 5 h unter Rückfluß erhitzt. Die Lösung wurde auf Raumtemperatur abgekühlt und Dioxan im Vakuum entfernt. Der Rückstand wurde mit wenig kaltem Wasser versetzt. Die wässrige Phase wurde mit konz. HCl auf pH 3 gestellt und der gebildete Niederschlag wurde abfiltriert.

Ausbeute: 7,54 g (95% d. Th.), logP¹⁾ (HCOOH) 0,50
¹H-NMR((CD₃)₂SO): 7,55 (m, 1H), 8,08 (s, 1H), 8,28 (m, 1H), 8,57 (m, 1H), 8,98 (s, 1H), 9,13 (m, 1H) ppm.

### Stufe 3: N-(Dimethylsulfamoyl)-1-(pyridin-3-yl)-1H-pyrazol-4-carboxamid

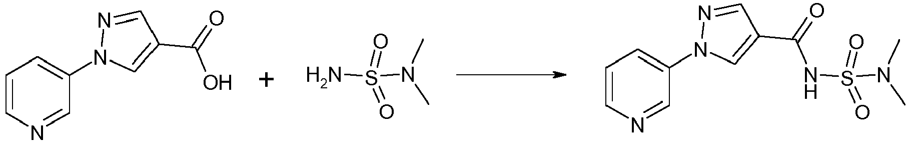

0,250 g (1,32 mmol) 1-(Pyridin-3-yl)-1H-pyrazol-4-carbonsäure wurden in 5 mL Tetrahydrofuran vorgelegt und mit 0,43 mL (1,98 mmol) N,N'-Carbonyldiimidazol versetzt. Die Mischung wurde 1 h unter Rückfluß erhitzt. 0,246 g (1,98 mmol) N,N-Dimethylsulfonamid wurden in 3 mL Tetrahydrofuran gelöst und bei Raumtemperatur zu der ersten Lösung zugetropft. Die Mischung wurde 10 min bei Raumtemperatur gerührt. Nach Zugabe von 0,30 mL (1,98 mmol) 1,8-Diazabicyclo(5.4.0)undec-7-en wurde die Mischung 12 h bei Raumtemperatur gerührt. Das Lösungsmittel wurden am Rotationsverdampfer entfernt und der Rückstand mit Wasser versetzt. Die wässrige Phase wurde mit Dichlormethan extrahiert. Die organische Phase wurde verworfen und die wässrige Phase mit konzentriertem HCl angesäuert. Der gebildete Niederschlag wurde abgesaugt.
Ausbeute: 0,120 g (30% d. Th.), logP¹⁾ (HCOOH) 1,03
¹H-NMR((CD₃)₂SO): 2,89(s,6H),7,58-7,62(m,1H),8,24-8,27(m,1H),8,38(s,1H),8,60-8,62(m,1H),9,10-9,11(m,1H),9,22(s,1H) ppm.

| **Verbindungsnummer** | **Strukturen** | **logP¹⁾ (HCOOH)** | **NMR Daten** |
|---|---|---|---|
| 4 (Beispiel D) | 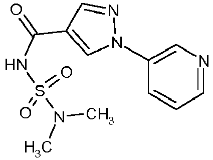 | 1,03 | ¹H-NMR((CD₃)₂SO): 2,89(s,6H), 7,58-7,62(m,1H), 8,24-8,27(m,1H), 8,38(s,1H), 8,60-8,62(m,1H), 9,10-9,11(m,1H), 9,22(s,1H) ppm |
| 6 | 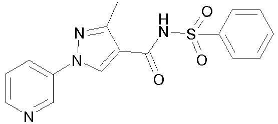 | 1,71 | ¹H-NMR ((CD₃)₂SO): 2,34(s,3H), 7,57-7,72(m,4H), 7,98-8,00(m,2H), 8,13-8,17(m,1H), 8,56-8,58(m,1H), 9,00-9,01(m,1H), 9,14(s,1H), 12,1(s,1H) |
| 7 | 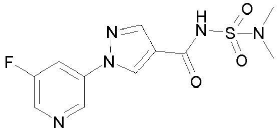 | 1,42 | ¹H-NMR ((CD₃)₂SO): 2,88(s,6H), 8,26-8,30(m,1H), 8,40(s,1H), 8,63-8,64(m,1H), 9,03(s,1H), 9,26(s,1H), 11,72(s,1H) |
| 8 | 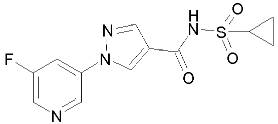 | 1,28 | ¹H-NMR ((CD₃)₂SO): 1,09-1,19(m,4H), 3,08-3,14(m,1H), 8,28-8,31(m,1H), 8,41(s,1H), 8,64-8,65(m,1H), 9,04-9,05(m,1H), 9,28-9,29(m,1H), 12,02(s,1H) |
| 9 | 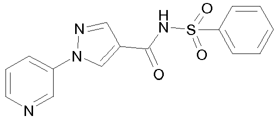 | 1,39 | ¹H-NMR ((CD₃)₂SO): 7,54-7,66(m,4H), 7,95-7,97(m,2H), 8,20-8,24(m,2H), 8,56-8,58(m,1H), 9,04(s,1H), 9,07-9,08(m,1H) |
| 11 | 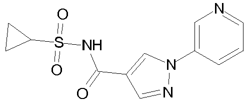 | 0,83 | |
| 12 | 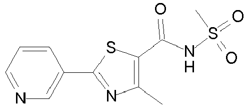 | 0,54 | |
| 13 | 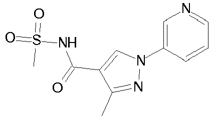 | 0,81 | |
| 15 | 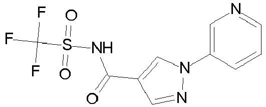 | 0,67 | |
| 16 | 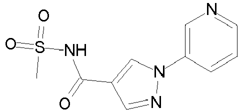 | 0,41 | |
| 17 | 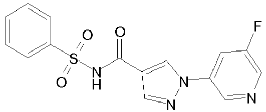 | 1,81 | |
| 18 | 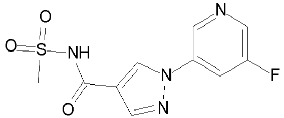 | 0,9 | |
| 19 | 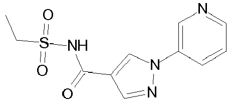 | 0,69 | |
| 20 | 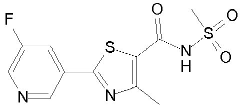 | 1,08 | |
| 21 | 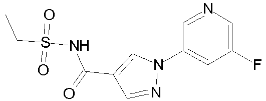 | 1,24 | |

### 1) Methodenbeschreibung zur Bestimmung der logP Werte (Ameisensäure Methode)

Die Bestimmung der in der Tabelle angegebenen logP-Werte erfolgte gemäß EEC-Directive 79/831 Annex V.A8 durch HPLC (High Performance Liquid Chromato-graphy) an einer Phasenumkehrsäule (C 18). Temperatur: 55°C.

Eluenten für die Bestimmung im sauren Bereich (pH 3,4):
Eluenat A: Acetonitril + 1 ml Ameisensäure/Liter. Eluent B: Wasser + 0,9 ml Ameisensäure/Liter.
Gradient: von 10% Eluent A / 90% Eluent B bis 95% Eluent A / 5% Eluent B in 4,25 min.

Die Eichung erfolgte mit unverzweigten Alkan-2-onen (mit 3 bis 16 Kohlen-stoff-atomen), deren logP-Werte bekannt sind (Bestimmung der logP-Werte anhand der Retentionszeiten durch lineare Interpolation zwischen zwei aufeinander folgenden Alkanonen). Die lambda-max-Werte wurden an Hand der UV-Spektren von 200 nm bis 400 nm in den Maxima der chromatographischen Signale ermittelt.

### 2) Messung der NMR-Spektren

### Die NMR-Spektren wurden

a) mit einem Bruker Avance 400, ausgestattet mit einem Durchflussprobenkopf (60 µl Volumen), bestimmt. Als Lösungsmittel wurden CD₃CN oder d₆-DMSO verwendet, wobei als Referenz Tetramethylsilan (0.00 ppm) eingesetzt wurde.
b) mit einem Bruker Avance II 600 bestimmt. Als Lösungsmittel wurden CD₃CN oder d₆-DMSO verwendet, wobei als Referenz Tetramethylsilan (0.00 ppm) eingesetzt wurde.

Die Aufstpaltung der Signale wurde wie folgt beschrieben: s (Singulett), d (Duplett), t (Triplett), q (Quartett), quin (Quintett), m (Multiplett).

**Myzus-Test (Spritzbehandlung)**

| | | |
|---|---|---|
| Lösungsmittel: | 78 | GewichtsteileAceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100% bei einer Aufwandmenge von 500g/ga: 7, 6

Bei diesem Test zeigten z. B. die folgenden Verbindungen der Herstellungsbeispiele Wirkung von 90% bei einer Aufwandmenge von 500g/ga: 4, 8

## Patentansprüche

1. Verbindungen der Formel (I) 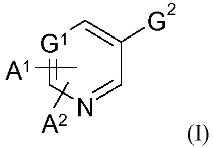 worin
A¹ für Wasserstoff, Halogen oder Cyano steht,
A² für Wasserstoff steht,
G¹ für N oder C-A¹ steht,
G² für einen Rest aus der Reihe 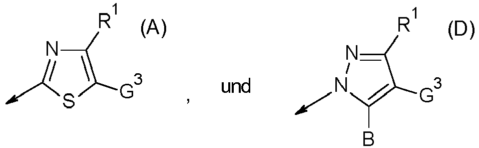 steht, worin der Pfeil die Bindung zum benachbarten Ring markiert,
R¹ für Wasserstoff oder C₁-C₄-Alkyl steht,
B für Wasserstoff steht,
G³ für den Rest 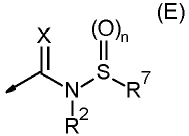 steht, worin der Pfeil die Bindung zu G² markiert,
X für Sauerstoff steht,
n für 2 steht,
R² für einen Rest aus der Reihe Wasserstoff, C₁-C₄-Alkyl und C₁-C₄-Alkoxy-C₁-C₄-alkyl, gegebenenfalls durch Halogen substituiertes C₁-C₄-Alkylcarbonyl, gegebenenfalls durch Halogen substituiertes C₁-C₄-Alkoxycarbonyl, gegebenenfalls durch Halogen, C₁-C₄-Alkyl, C₁-C₄-Alkoxy, C₁-C₄-Halogenalkyl und Cyano substituiertes C₃-C₆-Cycloalkylcarbonyl, oder für ein Kation, wie beispielsweise ein ein- oder zweiwertiges Metallion oder ein gegebenenfalls durch C₁-C₄-Alkyl oder Aryl-C₁-C₄-alkyl substituiertes Ammonium-Ion steht,
R⁷ für einen Rest aus der Reihe jeweils gegebenenfalls durch Halogen, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl und C₁-C₄-Halogenalkylsulfonyl substituiertes C₁-C₄-Alkyl, C₂-C₄-Alkenyl, C₂-C₄-Alkinyl, jeweils gegebenenfalls durch Halogen, C₁-C₄-Alkyl, C₁-C₄-Halogenalkyl, C₁-C₄-Alkoxy, C₁-C₄-Halogenalkoxy substituiertes C₃-C₆-Cycloalkyl, C₃-C₆-Cycloalkyl-C₁-C₄-alkyl, C₃-C₄-Cycloalkenyl, in welchen die Ringe mindestens ein Heteroatom aus der Reihe Schwefel, Sauerstoff (wobei Sauerstoffatome nicht unmittelbar benachbart sein dürfen) und Stickstoff enthalten können, jeweils gegebenenfalls durch Halogen, Cyano (auch im Alkylteil), Nitro, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl, Amino, C₁-C₄-Alkylamino, Di(C₁-C₄-alkyl)amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₃-C₆-Cycloalkyl-C₁-C₄-alkyl, C₁-C₄-Alkylcarbonyl, C₁-C₄-Alkoxycarbonyl oder Aminocarbonyl substituiertes Aryl, Heteroaryl, Aryl-C₁-C₄-alkyl und Heteroaryl-C₁-C₄-alkyl oder für NR'R" steht, worin R' und R" unabhängig voneinander für einen Rest aus der Reihe Wasserstoff und C₁-C₄-Alkyl stehen,
worin Halogen ausgewählt ist aus der Reihe Fluor, Chlor, Brom und Iod, Aryl (auch als Teil einer größeren Einheit, wie beispielsweise Arylalkyl) ausgewählt ist aus der Reihe Phenyl, Naphthyl, Anthryl, Phenanthrenyl und Hetaryl (auch als Teil einer größeren Einheit, wie beispielsweise Hetarylalkyl) ausgewählt ist aus der Reihe Pyrimidyl, Oxadiazolyl, Oxazolyl, Pyrazinyl, Imidazolyl, Thiazolyl und Furanyl,
sowie Salze und N-Oxide der Verbindungen der Formel (I).

2. Verbindungen der Formel (I) gemäß Anspruch 1, worin
A¹ für einen Rest aus der Reihe Wasserstoff, Fluor und Chlor steht,
A² für Wasserstoff steht,
G¹ für einen Rest aus der Reihe N, C-H, C-F und C-Cl steht,
G² für einen Rest aus der Reihe 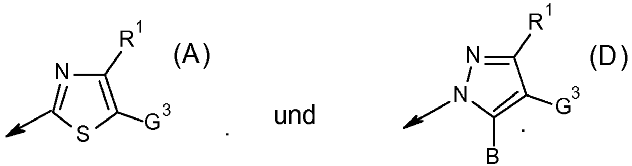 steht, worin der Pfeil die Bindung zum benachbarten Ring markiert,
R¹ für Wasserstoff oder Methyl steht,
B für Wasserstoff steht,
G³ für den Rest 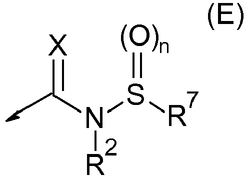 steht, worin der Pfeil die Bindung zu G² markiert,
X für Sauerstoff steht,
n für 2 steht,
R² für einen Rest aus der Reihe Wasserstoff, Methyl, Ethyl, CH₂OCH₃, CH₂OCH₂CH₃, COCH₃, COCH₂CH₃, Cyclopropyl, Na⁺, K⁺ und ⁺NMe₄ steht und
R⁷ für einen Rest aus der Reihe Methyl, Ethyl, i-Propyl, CF₃, CHF₂, CH₂F, CH₂CF₃, Cyclopropyl, Dimethylamino, Diethylamino, Phenyl und Benzyl steht.

3. Mittel, **gekennzeichnet durch** einen Gehalt von mindestens einer Verbindung der Formel (I) gemäß Anspruch 1 oder 2 und üblichen Streckmitteln und/oder oberflächenaktiven Substanzen.

4. Verfahren zum nicht therapeutischen Bekämpfen von Schädlingen **dadurch gekennzeichnet, dass** man eine Verbindung der Formel (I) gemäß Anspruch 1 oder 2 oder ein Mittel gemäß Anspruch 3 auf die Schädlinge und/oder ihren Lebensraum einwirken lässt.

5. Verwendung von Verbindungen der Formel (I) gemäß Anspruch 1 oder 2 oder von Mitteln gemäß Anspruch 3 zum nicht therapeutischen Bekämpfen von Schädlingen.

## Claims

1. Compounds of the formula (I) 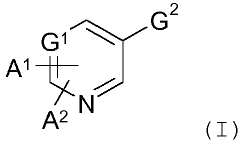 in which
A¹ is hydrogen, halogen or cyano,
A² is hydrogen,
G¹ is N or C-A¹,
G² is a radical from the group of 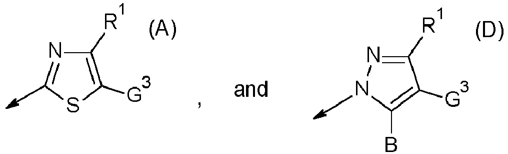 in which the arrow marks the bond to the adjacent ring,
R¹ is hydrogen or C₁-C₄-alkyl,
B is hydrogen,
G³ is the radical 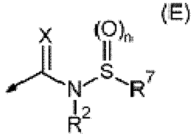 in which the arrow marks the bond to G²,
X is oxygen,
n is 2,
R² is a radical from the group of hydrogen, C₁-C₄-alkyl and C₁-C₄-alkoxy-C₁-C₄-alkyl, optionally halogen-substituted C₁-C₄-alkylcarbonyl, optionally halogen-substituted C₁-C₄-alkoxycarbonyl, optionally halogen-, C₁-C₄-alkyl-, C₁-C₄-alkoxy-, C₁-C₄-haloalkyl- and cyano-substituted C₃-C₆-cycloalkylcarbonyl, or a cation, for example a mono- or divalent metal ion or an optionally C₁-C₄-alkyl- or aryl-C₁-C₄-alkyl-substituted ammonium ion,
R⁷ is a radical from the group of in each case optionally halogen-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl- and C₁-C₄-haloalkylsulphonyl-substituted C₁-C₄-alkyl, C₂-C₄-alkenyl, C₂-C₄-alkynyl, in each case optionally halogen-, C₁-C₄-alkyl-, C₁-C₄-haloalkyl-, C₁-C₄-alkoxy-, C₁-C₄-haloalkoxy-substituted C₃-C₆-cycloalkyl, C₃-C₆-cycloalkyl-C₁-C₄-alkyl and C₃-C₄-cycloalkenyl, in which the rings may contain at least one heteroatom from the group of sulphur, oxygen (where oxygen atoms must not be immediately adjacent) and nitrogen, in each case optionally halogen-, cyano- (including in the alkyl moiety), nitro-, C₁-C₄-alkyl-, C₁-C₄-haloalkyl-, C₃-C₆-cycloalkyl-, C₁-C₄-alkoxy-, C₁-C₄-haloalkoxy-, C₁-C₄-alkylthio-, C₁-C₄-haloalkylthio-, C₁-C₄-alkylsulphinyl-, C₁-C₄-haloalkylsulphinyl-, C₁-C₄-alkylsulphonyl-, C₁-C₄-haloalkylsulphonyl-, amino-, C₁-C₄-alkylamino-, di (C₁-C₄-alkyl)amino-, C₁-C₄-alkylcarbonylamino-, C₁-C₄-alkoxycarbonylamino-, C₁-C₄-alkoxy-C₁-C₄-alkyl-, C₁-C₄-haloalkoxy-C₁-C₄-alkyl-, C₂-C₄-alkenyl-, C₂-C₄-alkynyl-, C₃-C₆-cycloalkyl-C₁-C₄-alkyl-, C₁-C₄-alkylcarbonyl-, C₁-C₄-alkoxycarbonyl- or aminocarbonyl-substituted aryl, heteroaryl, aryl-C₁-C₄-alkyl and heteroaryl-C₁-C₄-alkyl, or NR'R" in which R' and R" are each independently a radical from the group of hydrogen and C₁-C₄-alkyl,
in which halogen is selected from the group of fluorine, chlorine, bromine and iodine, aryl (including as part of a larger unit, for example arylalkyl) is selected from the group of phenyl, naphthyl, anthryl, phenanthrenyl, and hetaryl (including as part of a larger unit, for example hetarylalkyl) is selected from the group of pyrimidyl, oxadiazolyl, oxazolyl, pyrazinyl, imidazolyl, thiazolyl and furanyl,
and salts and N-oxides of the compounds of the formula (I).

2. Compounds of the formula (I) according to Claim 1, in which
A¹ is a radical from the group of hydrogen, fluorine and chlorine,
A² is hydrogen,
G¹ is a radical from the group of N, C-H, C-F and C-Cl,
G² is a radical from the group of 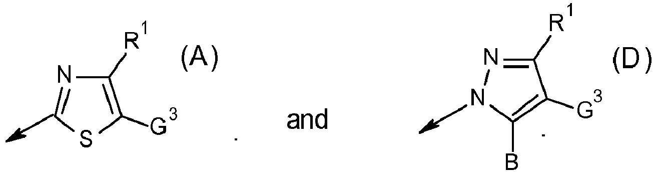 in which the arrow marks the bond to the adjacent ring,
R¹ is hydrogen or methyl,
B is hydrogen,
G³ is the radical 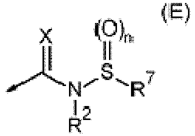 in which the arrow marks the bond to G²,
X is oxygen,
n is 2,
R² is a radical from the group of hydrogen, methyl, ethyl, CH₂OCH₃, CH₂OCH₂CH₃, COCH₃, COCH₂CH₃, cyclopropyl, Na⁺, K⁺ and ⁺NMe₄, and
R⁷ is a radical from the group of methyl, ethyl, i-propyl, CF₃, CHF₂, CH₂F, CH₂CF₃, cyclopropyl, dimethylamino, diethylamino, phenyl and benzyl.

3. Composition, **characterized by** a content of at least one compound of the formula (I) according to Claim 1 or 2 and customary extenders and/or surfactants.

4. Method for non-therapeutic control of pests, **characterized in that** a compound of the formula (I) according to Claim 1 or 2 or a composition according to Claim 3 is allowed to act on the pests and/or their habitat.

5. Use of compounds of the formula (I) according to Claim 1 or 2 or of compositions according to Claim 3 for non-therapeutic control of pests.

## Revendications

1. Composés de formule (I) 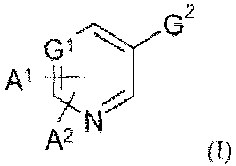 dans laquelle
A¹ représente hydrogène, halogène ou cyano,
A² représente hydrogène,
G¹ représente N ou C-A¹,
G² représente un radical de la série constituée par 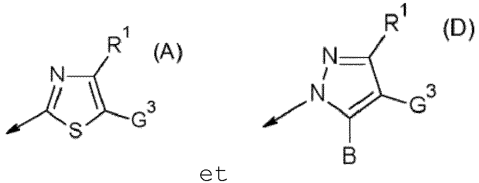
la flèche marquant la liaison au cycle voisin,
R¹ représente hydrogène ou alkyle en C₁-C₄,
B représente hydrogène,
G³ représente le radical 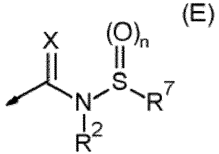
la flèche marquant la liaison à G²,
X représente oxygène,
n représente 2,
R² représente un radical de la série constituée par hydrogène, alkyle en C₁-C₄ et alcoxy en C₁-C₄-alkyle en C₁-C₄, alkylcarbonyle en C₁-C₄ éventuellement substitué par halogène, alcoxycarbonyle en C₁-C₄ éventuellement substitué par halogène, cycloalkylcarbonyle en C₃-C₆ éventuellement substitué par halogène, alkyle en C₁-C₄, alcoxy en C₁-C₄, halogénoalkyle en C₁-C₄ et cyano, ou un cation, tel que par exemple un ion métallique mono- ou bivalent ou un ion ammonium éventuellement substitué par alkyle en C₁-C₄ ou aryl-alkyle en C₁-C₄,
R⁷ représente un radical de la série constituée par alkyle en C₁-C₄, alcényle en C₂-C₄, alcynyle en C₂-C₄, chacun éventuellement substitué par halogène, alcoxy en C₁-C₄, halogénoalcoxy en C₁-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄ et halogénoalkylsulfonyle en C₁-C₄ ; cycloalkyle en C₃-C₆, cycloalkyle en C₃-C₆-alkyle en C₁-C₄, cycloalcényle en C₃-C₄, chacun éventuellement substitué par halogène, alkyle en C₁-C₄, halogénoalkyle en C₁-C₄, alcoxy en C₁-C₄, halogénoalcoxy en C₁-C₄, dans lesquels les cycles peuvent contenir au moins un hétéroatome de la série constituée par soufre, oxygène (des atomes d'oxygène ne pouvant pas être directement voisins) et azote ; aryle, hétéroaryle, aryl-alkyle en C₁-C₄ et hétéroaryl-alkyle en C₁-C₄, chacun éventuellement substitué par halogène, cyano (également dans la partie alkyle), nitro, alkyle en C₁-C₄, halogénoalkyle en C₁-C₄, cycloalkyle en C₃-C₆, alcoxy en C₁-C₄, halogénoalcoxy en C₁-C₄, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄, amino, alkylamino en C₁-C₄, di(alkyle en C₁-C₄)amino, alkylcarbonylamino en C₁-C₄, alcoxycarbonylamino en C₁-C₄, alcoxy en C₁-C₄-alkyle en C₁-C₄, halogénoalcoxy en C₁-C₄-alkyle en C₁-C₄, alcényle en C₂-C₄, alcynyle en C₂-C₄, cycloalkyle en C₃-C₆-alkyle en C₁-C₄, alkylcarbonyle en C₁-C₄, alcoxycarbonyle en C₁-C₄ ou aminocarbonyle ; ou NR'R", R' et R" représentant indépendamment l'un de l'autre un radical de la série constituée par hydrogène et alkyle en C₁-C₄,
l'halogène étant choisi dans la série constituée par fluor, chlore, brome et iode, l'aryle (également en tant que partie d'une unité plus grande, telle que par exemple arylalkyle) étant choisi dans la série constitué par phényle, naphtyle, anthryle, phénanthrényle, et l'hétaryle (également en tant que partie d'une unité plus grande, telle que par exemple hétarylalkyle) étant choisi dans la série constituée par pyrimidyle, oxadiazolyle, oxazolyle, pyrazinyle, imidazolyle, thiazolyle et furanyle, ainsi que les sels et N-oxydes des composés de formule (I).

2. Composés de formule (I) selon la revendication 1, dans lesquels
A¹ représente un radical de la série constituée par hydrogène, fluor et chlore,
A² représente hydrogène,
G¹ représente un radical de la série constituée par N, C-H, C-F et C-Cl,
G² représente un radical de la série constituée par 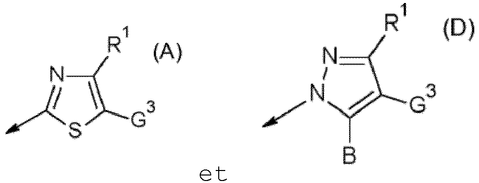 la flèche marquant la liaison au cycle voisin,
R¹ représente hydrogène ou méthyle,
B représente hydrogène,
G³ représente le radical 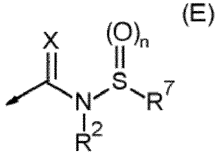
la flèche marquant la liaison à G²,
X représente oxygène,
n représente 2,
R² représente un radical de la série constituée par hydrogène, méthyle, éthyle, CH₂OCH₃, CH₂OCH₂CH₃, COCH₃, COCH₂CH₃, cyclopropyle, Na⁺, K⁺ et ⁺NMe₄, et
R⁷ représente un radical de la série constituée par méthyle, éthyle, i-propyle, CF₃, CHF₂, CH₂F, CH₂CF₃, cyclopropyle, diméthylamino, diéthylamino, phényle et benzyle.

3. Agent, **caractérisé par** une teneur en au moins un composé de formule (I) selon la revendication 1 ou 2 et des extendeurs et/ou substances tensioactives usuels.

4. Procédé de lutte non thérapeutique contre des nuisibles, **caractérisé en ce qu'**un composé de formule (I) selon la revendication 1 ou 2 ou un agent selon la revendication 3 est laissé agir sur les nuisibles et/ou leur habitat.

5. Utilisation de composés de formule (I) selon la revendication 1 ou 2 ou d'agents selon la revendication 3 pour la lutte non thérapeutique contre des nuisibles.
